# DEMANDE DE BREVET EUROPEEN

(11) **EP 2 546 784 A1**
(43) Date de publication de la demande: **16.01.2013**
(21) Numéro de dépôt: 12176071.4
(22) Date de dépôt: 12.07.2012
(51) Int. Cl.: G06K 19/077, G06K 19/04, G06K 17/00, A44C 5/00, G09F 3/00

(54) **Procédé, système et bandeau de communication de données à partir de données cryptées dans un environnement donné**

(30) Priorité: 13.07.2011 FR 1156366
(71) Demandeur: Furcate, Guy, 82200 Moissac (FR)
(72) Inventeur: Furcate, Guy, 82200 Moissac (FR)
(74) Mandataire: Gevers France

(57) **Abrégé**

L'invention vise à proposer un support de données cryptées particulier, compatible avec une retransmission des données en clair directement accessible.

Plus précisément, un système de communication expresse (1) selon l'invention comporte au moins un bandeau souple (2), muni de données cryptées imprimées (10) sur un support (22) solidarisé au bandeau (2), au moins un cellulaire (3) apte à télécharger une application d'un serveur (5) par une liaison bidirectionnelle (50) via Internet (4), cette application étant munie de moyens numériques aptes à acquérir et traduire les données cryptées (10) en données en clair puis à retransmettre ces données à l'écran d'affichage (30) du cellulaire (3). Un détecteur (6) permet avantageusement de localiser rapidement le bandeau (2).

Application en particulier à la communication de données médicales.

## Description

### DOMAINE TECHNIQUE

L'invention se rapporte à un procédé, un système et un bandeau de communication expresse de données à partir de données cryptées accessibles dans un environnement relatif à un être vivant, en particulier - mais non exclusivement - de données médicales.

L'invention s'applique au domaine de la communication de données d'information en cas d'urgence, par exemple en cas d'accident ou d'évanouissement d'une personne physique.

La transmission d'information d'ordre médicale ou connexe ou autre dans ce contexte peut être conditionnée par l'identification rapide des données. Le domaine de l'invention se rapporte donc également aux moyens propres à faciliter une telle identification en liaison avec la structure de ce support.

### ÉTAT DE LA TECHNIQUE

II existe des supports configurés pour pouvoir intégrer des données et pouvoir être portés par les personnes qui sont concernées par ces données, par exemple des données médicales. Ces supports sont notamment des bracelets ou des badges et les données sont intégrées sous forme de codes à barres lus par des lecteurs appropriés.

De telles solutions sont par exemple décrites dans les documents de brevet US 5 423574 ou US 6 144 304.

Cependant ces solutions ne sont pas efficaces lorsqu'il s'agit de transmettre des données dans l'urgence : le support de données n'est pas toujours aisément accessible ni même visible, les secouristes ou autres intervenants ne sont pas en général équipés de lecteurs appropriés. De plus l'équipement, qui nécessite un ordinateur et des lecteurs appropriés, est encombrant et onéreux.

### EXPOSÉ DE L'INVENTION

L'invention vise à s'affranchir de ces inconvénients en proposant un support de données cryptées particulier, compatible avec une retransmission des données en clair directement accessible.

Plus précisément, la présente invention a pour objet un procédé de communication expresse de données à un destinataire à partir de données cryptées relatives à un être vivant et accessibles dans son environnement, dans lequel les données cryptées sont imprimées sur un bandeau souple. Le bandeau souple est agencé de manière amovible par serrage autour d'un équipement en extension situé dans un environnement rapproché de l'être vivant, par exemple sur un habit ou un accessoire portée. Les données cryptées sont imprimées sous la forme d'un code, en particulier d'un code QR, apte à être dupliqué par un cellulaire détenu par le destinataire. Une application préalablement téléchargée d'un serveur relié par Internet audit cellulaire lit le code acquis et le décrypte. L'application fournit alors les données décryptées, c'est-à-dire en clair, par affichage sur le cellulaire.

Un être vivant se rapporte aux personnes physiques et aux animaux, en particulier aux animaux domestiques. Leur environnement rapproché signifie une proximité immédiate, à savoir sur l'être vivant (poignet, cou, etc.), sur ses vêtements ou dans l'espace qui l'entoure à portée de sa main.

Selon une particularité avantageuse, une identification par ondes, en particulier en bande radiofréquence, du bandeau souple est effectuée pour permettre au destinataire de détecter ce bandeau souple de manière encore plus rapide car, suivant les circonstances, le bandeau souple peut s'être déplacé ou même détaché de l'accessoire ou de l'équipement.

L'invention se rapporte également à un système de communication expresse apte à mettre en oeuvre ce procédé. Un tel système comporte au moins un bandeau souple muni d'au moins un support sur lequel des données cryptées sont imprimées, au moins un cellulaire apte à télécharger et mettre en oeuvre une application d'un serveur par une liaison bidirectionnelle via Internet, cette application étant munie de moyens numériques aptes à acquérir et traduire les données cryptées en données en clair puis à retransmettre ces données à l'écran d'affichage du cellulaire, ainsi que, de manière avantageuse, au moins un détecteur radiofréquence apte à localiser une étiquette RFID intégrée dans le bandeau.

Le bandeau souple de l'invention se présente en particulier sous la forme d'un bandeau en matériau synthétique comportant deux faces, une face externe sur laquelle au moins un support des données cryptées imprimées est solidarisé, et une face interne recouverte d'une bande adhésive apte à permettre une adhésion repositionnable du bandeau sur lui-même et autour d'une extension donnée, par exemple une extension dans un équipement, un accessoire ou un élément de vêtement de la personne concernée.

Selon d'autres modes de réalisation préférés :
- le bandeau est également équipé d'une étiquette d'identification par ondes, en particulier par ondes sur une bande de radiofréquence, connue sous l'appellation anglaise de tag RFID, l'étiquette étant solidarisée sur la face externe du bandeau;
- le bandeau est également équipé d'un autre support sur lequel est imprimée une photographie qui est présumée être celle du porteur de bandeau : lorsque l'être concerné est une personne physique, la photographie pouvant alternativement être imprimée sur le même support que le code ; l'invention prévoit ainsi l'adjonction d'une photographie de la personne concernée sur le bandeau souple à proximité du code, pour que le destinataire puisse immédiatement identifier la personne porteuse du bandeau souple comme étant celle correspondant au code situé à proximité de la photographie ;
- un cache amovible peut recouvrir le(s)dit(s) support(s),
- la longueur du bandeau est déterminée pour qu'une portion d'extrémité du bandeau munie d'un support de photographie vienne recouvrir une autre portion d'extrémité sans support.

Le bandeau selon l'invention est plus particulièrement destiné à être enroulé autour d'une jugulaire de casque de moto ou de bicyclette, d'une courroie de sécurité de véhicule automobile, ou d'un passant de jupe ou de pantalon, une épaulette d'imperméable ou autre élément de vêtement porté par la personne concernée. L'invention se rapporte à de tels accessoires munis d'un tel bandeau.

### PRÉSENTATION DES FIGURES

D'autres données, caractéristiques et avantages de la présente invention apparaîtront à la lecture de la description non limitée qui suit, en référence aux figures annexées qui représentent, respectivement :
- la figure 1, une vue schématique globale d'un exemple de système de communication selon l'invention ;
- la figure 2, un diagramme de mise en oeuvre du système selon la figure 1, et
- les figures 3a à 3d, différentes vues d'un exemple de bandeau selon l'invention en configuration dépliée (figure 3a) et en coupe partielle (figure 3b), pliée autour d'une jugulaire de casque (figure 3b) et autour d'une ceinture de sécurité automobile (figure 3c).

### DESCRIPTION DÉTAILLÉE

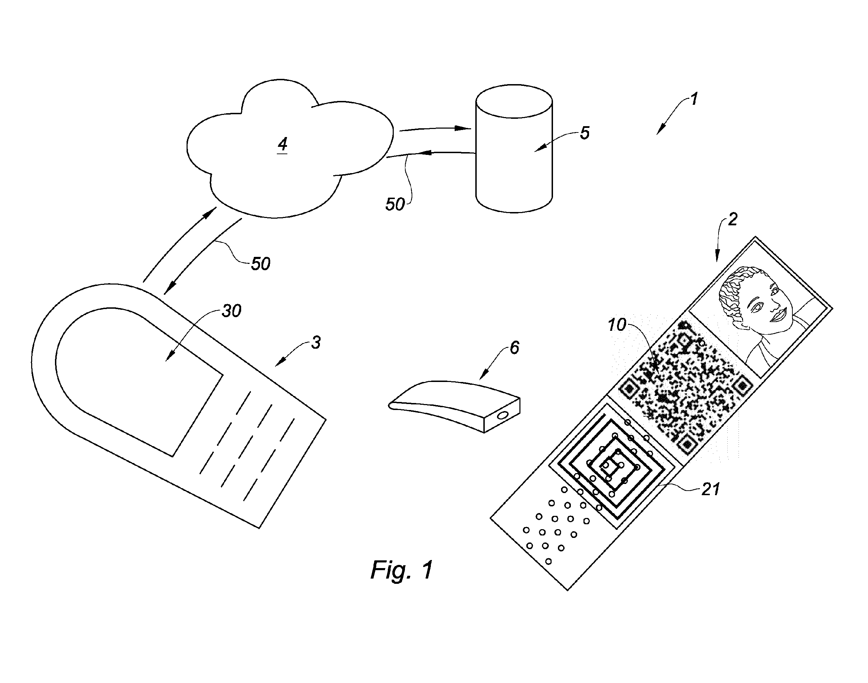

En référence à la vue schématique globale de la figure 1, un système de communication de données 1 selon l'invention comporte un bandeau souple 2, intégrant des données cryptées sous forme d'un code QR 10 dans l'exemple, et un téléphone cellulaire 3 relié via Internet 4 à un serveur 5. Les codes QR, qui peuvent être assimilés à des codes à barre en deux dimensions, permettent de coder un grand nombre d'informations à partir de texte ou de photographies.

Le serveur fournit par liaison Internet bidirectionnelle 50 une application logicielle de décryptage des codes QR au téléphone cellulaire qui retranscrit ensuite les données en clair sur son écran d'affichage 30. Le téléphone cellulaire 3 peut être de tout type, par exemple de type « smart-phone », muni d'un système d'exploitation apte à recevoir une telle application : « androïd », « windows », etc. De manière générique, le terme cellulaire est utilisé ci-après.

Le système comporte également, dans cet exemple de réalisation, un détecteur RFID 6 qui permet de détecter la présence d'une étiquette RFID 21 encapsulée dans le bandeau de données cryptées 2. De préférence, l'étiquette 21 est sans puce de manière à diminuer les coûts. Lorsque le détecteur est déclenché, il émet des ondes électromagnétiques afin de fournir de l'énergie à l'étiquette. Par courant induit, l'étiquette émet un signal qui est lu par le détecteur. L'échange s'opère dans des bandes de radiofréquences prédéfinies en technologie RFID. Dans le cas d'espèce, les radiofréquences utilisées sont autour de 13,5 MHz, permettant de procéder à des détections d'étiquettes à courte distance, ici jusqu'à environ 10 à 15 cm. L'étiquette peut être passive ou active afin de renforcer la puissance du signal.

Dans l'exemple de réalisation illustré, l'application concerne l'identification du profil médical de personnes accidentées, traumatisées, atteintes de la maladie d'Alzheimer et/ou en perte de conscience. Le bandeau dit « bandeau médical » de la personne concernée comporte des informations personnelles prioritaires dans le code: nom, prénoms, numéro de sécurité social, mutuelle, date de naissance, numéros de téléphone et email des personnes et organismes à contacter.

Des informations prioritaires sur le profil médical de cette personne sont également intégrées dans le code: groupe sanguin, maladies, principaux antécédents médicaux, allergies, lien url d'un site accessible via Internet qui stocke d'autres informations (prescriptions médicales actualisées, autres antécédents, photographies, don d'organe autorisé ou refusé, etc.). Toutes les informations sont cryptées par le codage QR. Pour réaliser ce code QR personnalisé, les renseignements fournis par la personne concernée sont préalablement collectés et triés sur des pages « CV MEDICAL INFO » du site Internet dédié.

La rapidité de détection du bandeau médical et donc du code QR peut permettre de gagner un temps précieux pour la survie de ces personnes. C'est pourquoi l'adjonction d'un moyen de détection du bandeau médical à courte distance est particulièrement avantageuse.

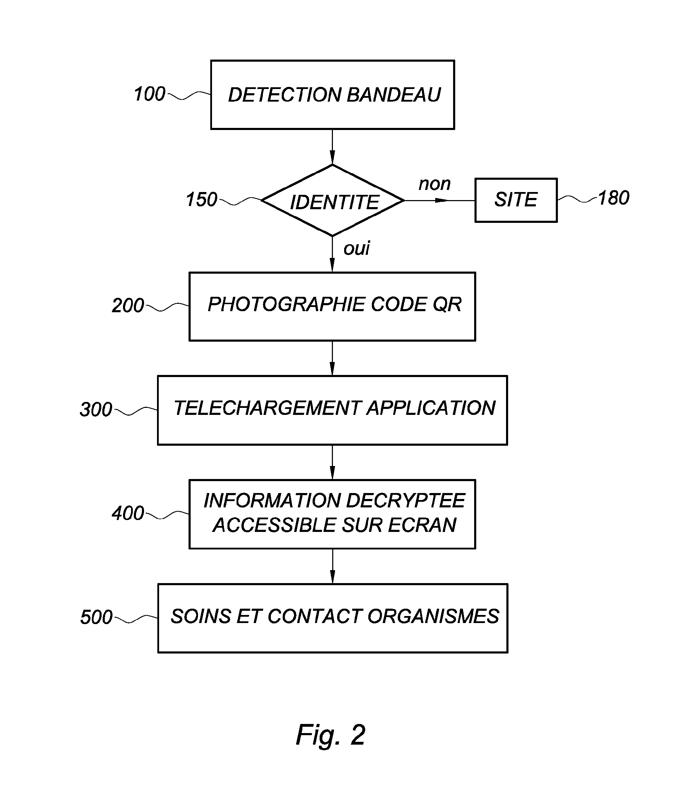

Les étapes successives de fourniture de données lisibles à un intervenant équipé du cellulaire décrit ci-dessus, à partir des données cryptées du bandeau médical sont maintenant décrites en référence au diagramme de la figure 2. L'intervenant secouriste détecte la présence du bandeau médical (étape 100) en passant son détecteur RFID sur toute la personne, à une distance d'environ 10 cm. Une fois le bandeau détecté, l'intervenant procède à l'identification préalable de la personne par comparaison entre la photographie sur le bandeau et le visage de la personne à traiter (étape 150).

Si la personne ne correspond pas à la photographie (« non » sur le diagramme), l'intervenant prend une photo de cette personne avec son téléphone cellulaire et la transmet au serveur qui, en liaison avec le site d'information décrit ci-dessus, compare les photographies et fournit les informations médicales disponibles relatives à la personne réellement concernée (étape 180).

Lorsque la personne est bien identifiée (« oui » sur le diagramme), l'intervenant photographie le code QR du bandeau grâce à son cellulaire (étape 200). L'intervenant télécharge alors l'application (étape 300) qui traduit en clair les informations médicales initialement codées grâce à un scanner intégré. Enfin, l'intervenant lit les informations décryptées transmises sur l'écran du cellulaire (étape 400) et contacte les organismes habilités afin de fournir les informations pertinentes (étape 500). Il prodigue alors les soins en fonction des informations reçues.

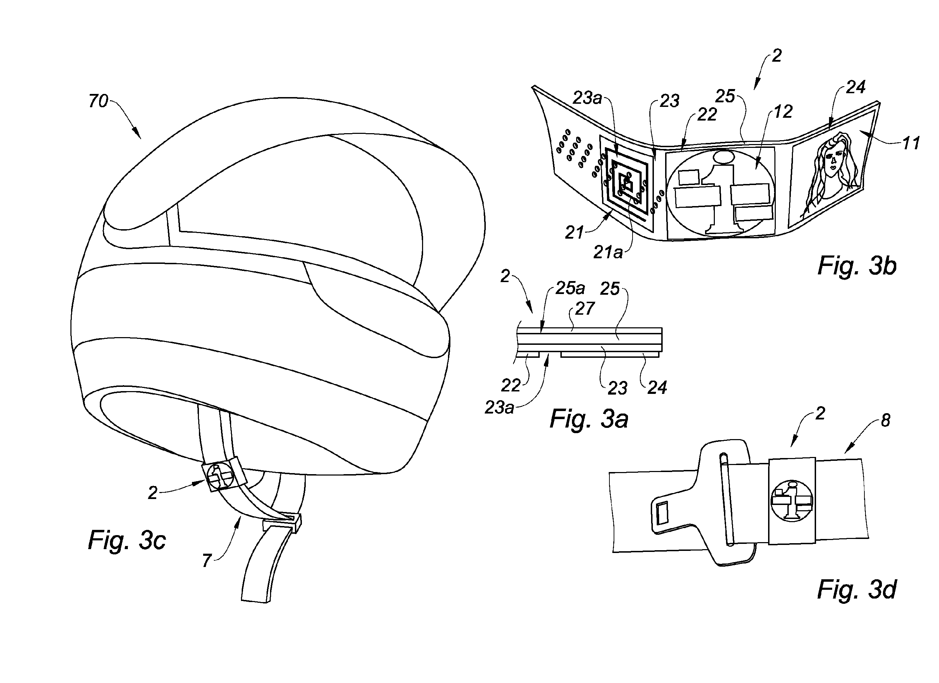

Le bandeau médical 2 est plus précisément illustré en configuration dépliée sur la figure 3a et vue en coupe partielle sur la figure 3b. II se présente sous la forme d'une bande longitudinale en matière synthétique souple. Le bandeau comporte une couche de revêtement en polyamide 23 sur une couche polyester 25 recouverte, sur sa face 25a dite face interne, d'une couche adhésive en forme de bande adhésive repositionnabie 27. L'étiquette RFID 21 avec son antenne en spirale 21a encapsulée est intégrée au bandeau 2. Globalement, l'épaisseur du bandeau ne dépasse pas environ 1 mm.

Des supports en PVC 22 et 24, sur lesquels sont imprimés le code QR et une photographie 11 de la personne concernée, sont collés sur la face dite externe 23a de la couche polyamide 23 du bandeau 2. Avantageusement, le code QR est recouvert d'un cache sous forme d'autocollant de confidentialité 12, aisément décollable. Sur cet autocollant une mention d'identification explicite peut être imprimée, par exemple « C.V. MEDICAL INFO ».

En utilisation, le bandeau 2 est replié sur lui-même de manière serrée, par exemple autour d'une jugulaire 7 de casque de motocyclette 70, comme illustré par l'exemple d'application de la figure 3c. La longueur du bandeau a été déterminée en fonction de la largeur de la jugulaire 7 pour que la portion d'extrémité du bandeau 2 munie du support de photographie 24 vienne recouvrir l'autre portion d'extrémité sans support, sur une face interne de la jugulaire 7, afin de conserver la photographie apparente.

Dans une autre application illustrée par la figure 3d, le bandeau 2 est replié autour d'une ceinture de sécurité 8 d'un véhicule automobile. Dans d'autres applications, le bandeau peut être replié autour d'un bracelet-montre ou équivalent, ou de tout accessoire de vêtement : passants de pantalon ou jupe, épaulettes, etc. II peut aussi directement servir de bracelet-montre ou d'accessoire.

L'invention n'est pas limitée aux exemples de réalisation décrits et représentés. Ainsi, le bandeau peut être apposé sur une vitre ou pare-brise de véhicule, ou encore sur un casque. II peut se présenter sous la forme d'une bande auto-agrippante de type « velcro ». L'étiquette RFID peut être encapsulée dans le support du code QR ou de la photographie.

La détection peut être réalisée par d'autres moyens que le couple détecteur / étiquette RFID. Par exemple, une détection optique, par exemple infrarouge, ou par ultrasons ou équivalent peut être mise en oeuvre.

En variante, le bandeau peut être définitivement fixé dans ou autour de tels accessoires ou équipements, ou peut encore être enroulé autour du poignet, de la cheville ou du cou de la personne concernée.

L'invention peut également s'appliquer à tout être vivant, par exemple un animal domestique.

## Revendications

1. Procédé de communication expresse de données à un destinataire à partir de données cryptées relatives à un être vivant et accessibles dans son environnement, dans lequel les données cryptées sont imprimées sur un bandeau souple (2), **caractérisé en ce que** le bandeau souple (2) est agencé de manière amovible par serrage autour d'un équipement en extension (7, 8) situé dans un environnement rapproché de l'être vivant, **en ce qu'**il est en matériau synthétique et comporte une face externe (23a), sur laquelle au moins un support (22, 24) de données cryptées imprimées (10) est solidarisé, et une face interne (25a) recouverte d'une bande adhésive (27) apte à permettre une adhésion repositionnable du bandeau (2) sur lui-même et autour d'une extension donnée, **en ce que** les données cryptées sont imprimées sous la forme d'un code, en particulier d'un code QR (10), apte à être acquis par un cellulaire (3) détenu par le destinataire, et **en ce qu'**une application, préalablement téléchargée (50) d'un serveur (5) relié par Internet audit cellulaire (3), lit le code acquis (10) et le décrypte, l'application fournissant ensuite les données décryptées par affichage (30) sur le cellulaire (3).

2. Procédé selon la revendication 1, dans lequel une identification par ondes (21) du bandeau souple (2), en particulier en bande radiofréquence, est effectuée.

3. Système de communication expresse (1) apte à mettre en oeuvre le procédé selon l'une quelconque des revendications 1 à 2, **caractérisé en ce qu'**il comporte au moins un bandeau souple (2) muni d'au moins un support (22) sur lequel des données cryptées sont imprimées (10), au moins un cellulaire (3) apte à télécharger et mettre en oeuvre une application d'un serveur (5) par une liaison bidirectionnelle (50) via Internet (4), cette application étant munie de moyens numériques aptes à acquérir et traduire les données cryptées (10) en données en clair puis à retransmettre ces données à l'écran d'affichage (30) du cellulaire (3).

4. Système de communication expresse selon la revendication précédente, dans lequel au moins un détecteur radiofréquence (6) est apte à localiser une étiquette RFID (21) intégrée dans le bandeau (2).

5. Bandeau souple apte à être utilisé dans un système selon l'une des revendications 3 ou 4, **caractérisé en ce qu'**une étiquette d'identification par ondes (21), en particulier par ondes de radiofréquence, est solidarisée sur la face externe (23a) du bandeau (2).

6. Bandeau souple selon la revendication 5, dans lequel une photographie (11) est imprimée sur un autre (24) ou le même (22) support solidarisé sur la face externe (23a) du bandeau (2).

7. Bandeau souple selon l'une des revendications 5 ou 6, dans lequel un cache amovible (12) recouvre le ou les supports (22, 24).

8. Bandeau souple selon l'une des revendications 5 ou 6, dans lequel la longueur du bandeau est déterminée pour qu'une portion d'extrémité du bandeau munie d'un support de photographie ou de support de données cryptées vienne recouvrir une autre portion d'extrémité sans support.
